(19) Europäisches Patentamt / European Patent Office / Office européen des brevets

(11) **EP 3 158 926 B1**

(12) **EUROPEAN PATENT SPECIFICATION**

(45) Date of publication and mention
of the grant of the patent:
**01.08.2018   Bulletin 2018/31**

(51) Int Cl.:
*A61B 5/021* (2006.01)     *A61B 5/0205* (2006.01)
*A61M 16/00* (2006.01)     *A61B 5/08* (2006.01)

(21) Application number: **16193277.7**

(22) Date of filing: **11.10.2016**

(54) **PULSE WAVE ANALYZER**

PULSWELLENANALYSATOR

ANALYSEUR D'ONDES DE POULS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **19.10.2015   JP 2015205535**

(43) Date of publication of application:
**26.04.2017   Bulletin 2017/17**

(73) Proprietor: **Nihon Kohden Corporation
Shinjuku-ku
Tokyo (JP)**

(72) Inventors:
• **Tanishima, Masami
Tokyo (JP)**
• **Sakai, Tomoyuki
Tokyo (JP)**
• **Mato, Takashi
Tokyo (JP)**

(74) Representative: **Hoffmann Eitle
Patent- und Rechtsanwälte PartmbB
Arabellastraße 30
81925 München (DE)**

(56) References cited:
**EP-A1- 2 198 776**     **EP-A1- 2 263 528**
**US-A1- 2005 245 830**

**Description**

BACKGROUND

The presently disclosed subject matter relates to an apparatus for analyzing a pulse wave which is acquired from a subject.

The respiratory variation of the arterial pressure may be an important index for knowing the circulatory dynamics in the subject. In the case where the circulatory blood volume in the subject is not sufficient, the value of the respiratory variation of the arterial pressure is large. In the case where the circulatory blood volume in the subject is sufficient, by contrast, the value of the respiratory variation of the arterial pressure is small.

JP-T-2011-511686 discloses an apparatus for measuring the pulse pressure variation (respiratory variation) in the respiratory cycle.

US 2005/245830 A1 describes monitoring a patient including acquiring a respiration waveform (e.g. CO2) and an arterial pressure waveform. Based on this data, a systolic pressure variation is calculated. This document also discloses that the respiration waveform that represents end expiration provides an approximation of apnea.

EP 2 198 776 A1 describes an apparatus able to determine whether or not a patient is breathing spontaneously.

EP 2 263 528 A1 describes that pulse pressure variation measurement is generally limited to mechanically ventilated patients and cannot be applied to spontaneously breathing patients. The display mode of the patient monitor is switched to no longer show the PPV value when spontaneous breathing is detected.

In the case where the respiration of the subject is assisted by using an artificial respirator, it is contemplated that the acquisition of the circulatory dynamics in the subject is effective for determining whether the respiration assistance using the artificial respirator is adequately performed or not.

Therefore, the inventors studied a method of acquiring the circulatory dynamics in the subject in whom the respiration is assisted by an artificial respirator, based on the respiratory variation. However, it has been often that, in the case where the respiratory variation is used as an index for knowing the circulatory dynamics in the subject, the accuracy of the respiratory variation which is calculated by using a related-art apparatus is low.

SUMMARY

The presently disclosed subject matter may provide a pulse wave analyzer in which the accuracy of the calculation of the respiratory variation that is an index for knowing the circulatory dynamics in the subject can be improved.

The pulse wave analyzer may comprise: a pulse wave acquirer which is configured to acquire a pulse wave of a subject; a respiration information acquirer which is configured to acquire respiration information of the subject; and an analyzing section which includes: a detector which, based on the respiration information, is configured to detect that spontaneous respiration is generated, and to detect an end-tidal phase of the spontaneous respiration; and an eliminator which is configured to eliminate a unit pulse wave that, in the pulse wave, is generated immediately after the end-tidal phase of the spontaneous respiration, from the pulse wave, wherein the analyzing section is configured to calculate a respiratory variation from the pulse wave from which the unit pulse wave has been eliminated.

BRIEF DESCRIPTION OF THE DRAWINGS

Fig. 1 is a diagram illustrating a pulse wave analyzer of an embodiment of the presently disclosed subject matter.
Fig. 2 is a view illustrating the respiratory variation which is calculated by the analyzer.
Fig. 3 is a view illustrating vital signs of the subject which are acquired by the analyzer.
Fig. 4 is a flowchart illustrating the operation of the analyzer.
Fig. 5 is a view illustrating an example of an analysis result which is displayed on a displaying section of the analyzer.
Fig. 6 is a view in which the respiratory variation calculated by the analyzer is compared with that calculated by a related-art calculation method.

DETAILED DESCRIPTION OF EXEMPLARY EMBODIMENTS

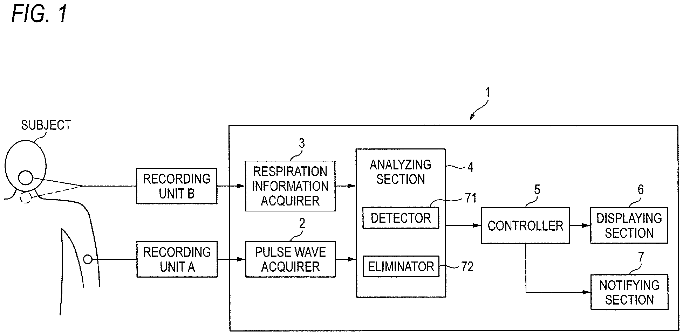

Hereinafter, an embodiment of the presently disclosed subject matter will be described in detail with reference to the drawings. Fig. 1 is a functional block diagram illustrating a pulse wave analyzer 1. The pulse wave analyzer 1 has a function of calculating the respiratory variation in a subject to whom an artificial respirator is attached. For example, the value indicating the respiratory variation may be selected from the PPV (Pulse Pressure Variation) indicating the change rate of the pulse pressure, the SPV (Systolic Pressure Variation) indicating the variation rate of the systolic pressure of the pulse pressure, the SVV (Stroke Volume Variation) indicating the change rate of the stroke volume, the PVI (Pleth Variability Index) indicating the variation rate of the pulse wave in the arterial oxygen saturation ($SpO_2$), and the like.

As illustrated in Fig. 1, the pulse wave analyzer 1 may include a pulse wave acquirer 2, a respiration information acquirer 3, an analyzing section 4, a controller 5, a displaying section 6, and a notifying section 7.

The pulse wave acquirer 2 is communicably connected to a recording unit A. The pulse wave acquirer 2 acquires the arterial pressure of the subject from the recording unit A, and produces the blood pressure waveform (pulse wave) from the arterial pressure. The pulse wave is configured by a plurality of continuous unit pulse waves. A unit pulse wave means a unit of pulse wave corresponding to one heart beat. The recording unit A is configured by, for example, a catheter or the like which is to be inserted into the blood vessel of the subject. As the recording unit A, alternatively, a blood pressure measurement cuff which is to be attached to an upper arm portion, an pulse oximeter in which a probe is attached to the finger tip or the ear, or the like may be used.

The respiration information acquirer 3 is communicably connected to a recording unit B. The respiration information acquirer 3 acquires the respiration information from the recording unit B. The recording unit B is configured by, for example, an artificial respirator which supplies and discharges (ventilates) the air through a tube attached to the subject, to assist the respiration of the subject. For example, the respiration information includes the cycle of forced ventilation which is controlled by the artificial respirator, timings when spontaneous respiration of the subject is generated, the respiratory rate in the subject, the concentration of carbon dioxide ($CO_2$ concentration) in the respiration of the subject, etc. The artificial respirator is configured so as to be able to assist also a subject in whom spontaneous respiration is generated. The artificial respirator has ventilation modes such as the A/C (Assist Control), the SIMV (Synchronized Intermittent Mandatory Ventilation), the PSV (Pressure Support Ventilation), and the CPAP (Continuous Positive Airway Pressure).

The analyzing section 4 calculates the value of the respiratory variation based on the pulse wave acquired from the subject. In the embodiment, the analyzing section 4 selects unit pulse waves by using the respiration information, and calculates the value of the respiratory variation. The analyzing section 4 may include a detector 71 and an eliminator 72.

The detector 71 detects that spontaneous respiration is generated in the subject, based on the respiration information. The detector 71 further detects the end-tidal phase of the detected spontaneous respiration.

The eliminator 72 identifies, in the pulse wave acquired from the subject, a unit pulse wave which is generated immediately after the end-tidal phase of spontaneous respiration of the subject. The eliminator 72 eliminates the identified unit pulse wave from the pulse wave of the subject.

The controller 5 controls the contents to be displayed on the displaying section 6, and those to be notified by the notifying section 7, and the like. The displaying section 6 displays analysis information of the pulse wave which is output from the analyzing section 4. The displaying section 6 is configured by, for example, a touch-panel liquid crystal display device. The notifying section 7 notifies of an abnormality of an analysis result. The notifying section 7 is configured by, for example, a speaker.

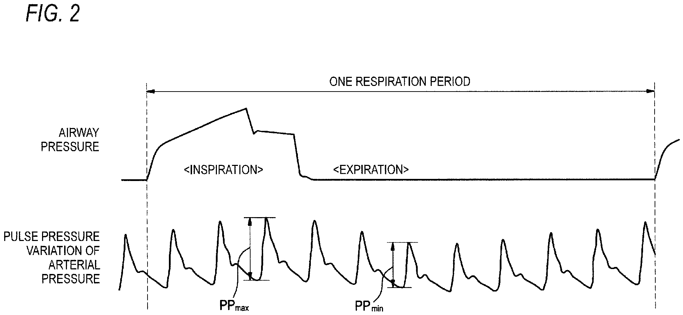

Next, the respiratory variation calculated by the analyzing section 4 will be described with reference to Fig. 2. The respiratory variation (in the embodiment, the PPV is used) is obtained from following Expression 1:

$$\mathrm{PPV} = (\mathrm{PPmax} - \mathrm{PPmin})/((\mathrm{PPmax} + \mathrm{PPmin})/2) \times 100\ [\%]$$

$$\ldots (\text{Expression 1}).$$

The respiratory variation which is obtained from the expression is a value which is obtained by dividing the difference between the maximum amplitude (PPmax) and the minimum amplitude (PPmin) of the pulse wave in one cycle of respiration, by the average of the maximum amplitude and the minimum amplitude of the pulse wave, and indicates the variation rate of the amplitude level of the pulse wave in one reciprocation cycle. The calculation is completed by computation on time-axis data, and obtains one variation rate per reciprocation cycle.

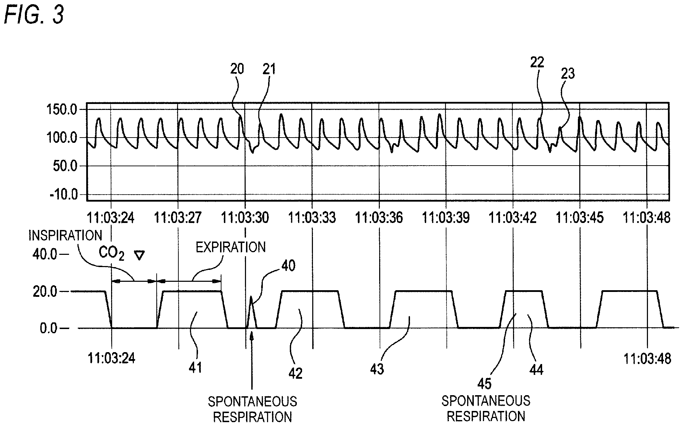

Next, vital signs acquired by the pulse wave acquirer 2 and the respiration information acquirer 3 will be described with reference to Fig. 3.

In the two graphs illustrated in Fig. 3, the upper graph indicates the pulse wave of the subject acquired by the pulse wave acquirer 2, and the lower graph indicates the $CO_2$ concentration of the subject acquired by the respiration information acquirer 3. The pulse wave acquirer 2 and the respiration information acquirer 3 are configured so that the timing when the pulse wave acquirer 2 acquires the pulse wave is synchronized with that when the respiration information acquirer 3 acquires the $CO_2$ concentration.

As indicated in the graph of the $CO_2$ concentration, the cycle of the forced ventilation which, in the embodiment, is controlled by the artificial respirator is set so as to be 2 seconds in inspiration, and 3 seconds in expiration. A waveform 40 is generated between expiration 41 and expiration 42. This indicates a change of the $CO_2$ concentration due to spontaneous respiration in the subject. The time period of the expiration 44 is shorter than 3 seconds. This indicates that the subject generates spontaneous respiration 45 during the expiration 44.

As indicated in the graph of the pulse wave, a unit pulse wave 21 in which the variation rate is large (the amplitude is low) with respect to the amplitude of the immediately preceding unit pulse wave 20 is generated in synchronization with the generation timing of the spontaneous respiration (waveform 40) in the subject. In accordance with the generation of the spontaneous respiration 45 in the subject, a unit pulse wave 23 in which the variation rate is large (the amplitude is low) with respect to the amplitude of the immediately preceding unit pulse wave 22 is generated after the expiration 44 ends. In the embodiment, among unit pulse waves in which the amplitude is varied by spontaneous respiration, arrhythmia, a change of the body posture, a body motion, or the like, a unit pulse wave in which the amplitude is varied by spontaneous respiration is detected and eliminated.

In the elimination of such a unit pulse wave, the inventors have paid attention on a phenomenon in which the amplitude of a pulse wave tends to be varied in inspiration immediately after the end-tidal phase of the respiration. Firstly, therefore, a unit pulse wave which is detected in the end-tidal phase was eliminated in inspiration phases immediately after all the end-tidal phases. In this case, however, even a unit pulse wave which is effective (which should not be eliminated) in calculation of the respiratory variation was eliminated, and the respiratory variation was not accurately calculated.

Next, the inventors have studied the elimination with consideration of generation/non-generation of spontaneous respiration in the subject. In the case where a subject to whom an artificial respirator is attached generates spontaneous respiration, the timing when inspiration assisted by the artificial respirator starts is delayed by the time period corresponding to the response time of the respirator, and therefore the intrathoracic pressure during inspiration is a negative pressure which is higher than that in a subject to whom an artificial respirator is not attached. When the intrathoracic pressure is negative, the blood is sucked into the pleural cavity, and accumulated therein. Therefore, the discharge amount is reduced at the timing, and the amplitude of a pulse wave occurring thereafter is varied. The lowering rate of the intrathoracic pressure is changed also by the strength of inspiration, and the amplitude of a pulse wave occurring thereafter is varied also by lowering of the intrathoracic pressure. Consequently, the inventors cause a unit pulse wave (for example, the unit pulse wave 23 illustrated in Fig. 3) in which, when spontaneous respiration is generated, the variation rate of the amplitude in inspiration immediately after the end-tidal phase of the spontaneous respiration is large, to be eliminated.

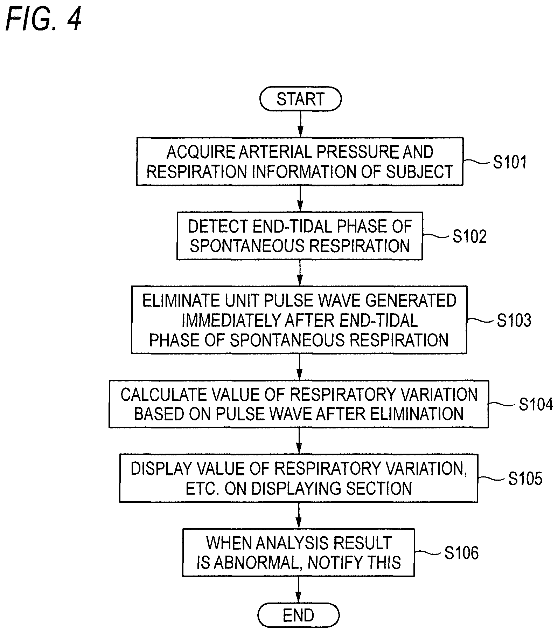

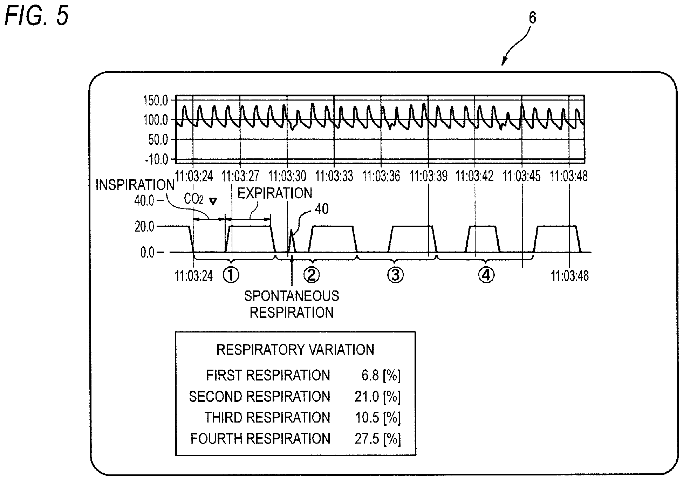

Next, the operation of the pulse wave analyzer 1 will be described with reference to Figs. 4 and 5.

As a preparatory step, a catheter (the recording unit A) is inserted into the blood vessel of the subject. An artificial respirator (the recording unit B) is previously attached to the subject.

When the operation of analyzing the pulse wave is started, the pulse wave acquirer 2 acquires the arterial pressure recorded by the catheter, from the catheter, and applies signal processing on the acquired arterial pressure to take out the blood pressure waveform (pulse wave) (step S101, the upper graph of Fig. 3) . Moreover, the respiration information acquirer 3 acquires the respiration information of the subject such as the $CO_2$ concentration (the lower graph of Fig. 3), the respiratory cycle, timings when spontaneous respiration is generated, and the respiratory rate, from the artificial respirator (step S101) .

The detector 71 of the analyzing section 4 acquires the respiration information from the respiration information acquirer 3, detects that the subject generates spontaneous respiration, and further detects the end-tidal phase of the generated spontaneous respiration (step S102).

The eliminator 72 acquires pulse waves from the pulse wave acquirer 2, and identifies the pulse wave which is acquired in synchronization with the end-tidal phase of the generated spontaneous respiration. In the identified pulse wave, the eliminator 72 identifies a unit pulse wave which is generated immediately after the end-tidal phase of the spontaneous respiration, and eliminates the identified unit pulse wave from pulse waves in one cycle of respiration (step S103).

The analyzing section 4 calculates the respiratory variation in each respiration cycle based on the pulse wave after the elimination processed in step S103 (step S104).

Then, the controller 5 causes the vital signs, and generation/non-generation and and generation timing of spontaneous respiration which are acquired by the pulse wave acquirer 2 and the respiration information acquirer 3 in step S101, the values of the respiratory variations in respective respiration cycles which are calculated in step S104, and the like to be displayed on the the display device of the displaying section 6 (step S105, Fig. 5).

In the case where the value of the calculated respiratory variation exceeds a predetermined threshold, the controller 5 causes the notifying section 7 to output, for example, a warning alarm (step S106).

The respiratory variation (for example, the PPV) is expected as an important parameter for transfusion management due to insufficiency of the circulatory blood volume. In the prior art, however, the respiratory variation is obtained with many restrictions, and under the specifications that it can be measured only during stable positive-pressure respiration which is caused by an artificial respirator, and in which arrhythmia and spontaneous respiration are not generated.

In, for example, an ICU (Intensive Care Unit), when the patient generates spontaneous respiration, by contrast, a ventilation mode of an artificial respirator in which the spontaneous respiration is used is frequently employed. Therefore, it is often that the value of the respiratory variation becomes unstable due to a unit pulse wave which is varied in accordance with the generation of spontaneous respiration.

According to the configuration of the presently disclosed subject matter, by contrast, a unit pulse wave which is generated immediately after the end-tidal phase of spontaneous respiration, i.e., a unit pulse wave which is discontinuously changed (the variation rate of the amplitude is large) can be eliminated in the calculation of the respiratory variation. In the case where the respiration of the subject is assisted by, for example, using an artificial respirator, even when the subject generates spontaneous respiration, therefore, it is possible to accurately obtain the respiratory variation which is an index for knowing the circulatory dynamics in the subject.

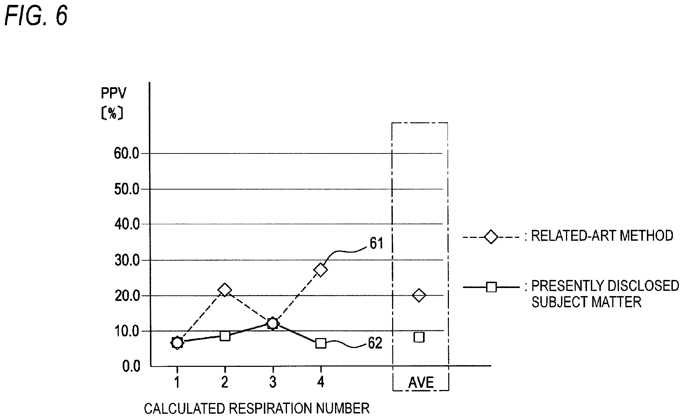

Fig. 6 is a graph in which the value of the respiratory variation calculated according to the presently disclosed subject matter is compared with that of the respiratory variation calculated by a related-art method. In Fig. 6, the abscissa indicates the sample number of one cycle of respiration in which the respiratory variation is calculated, and the ordinate indicates the value of the respiratory variation. The symbol denoted by the reference numeral 61 indicates the value of the respiratory variation that was calculated by the related-art calculation method in which all unit pulse waves were used irrespective of generation/non-generation of spontaneous respiration (a pulse wave was not eliminated). The symbol denoted by the reference numeral 62 indicates the value of the respiratory variation that was calculated by the presently disclosed subject matter where a unit pulse wave was eliminated in which the variation rate when spontaneous respiration is generated is large.

In the respiratory variation in one cycle of respiration in which a change of the body posture, a body motion, spontaneous respiration, arrhythmia, and the like do not occur in the subject (for example, Sample No. 1), the value of the respiratory variation calculated by the related-art method is approximately equal to that of the respiratory variation calculated by the presently disclosed subject matter. In the respiratory variation in one cycle of respiration in which spontaneous respiration occurs in the subject (for example, Sample No. 4), the value calculated by the related-art method is largely varied from the value of spontaneous respiration in Sample No. 1, but the value calculated by the presently disclosed subject matter is approximately equal to the value of spontaneous respiration in Sample No. 1. In the case of respiratory variation in one cycle of respiration in which a cough or a body motion occurs in the subject (for example, Sample No. 3), the value calculated by the related-art method is varied similarly with that of the respiratory variation calculated by the presently disclosed subject matter.

The above-described embodiment is a mere example for facilitating understanding of the presently disclosed subject matter, and is not intended to limit the presently disclosed subject matter. The presently disclosed subject matter may be adequately changed or improved without departing from the scope of the presently disclosed subject matter. It is obvious that equivalents are included within the technical scope of the presently disclosed subject matter.

Although, in the embodiment, for example, the eliminator 72 eliminates, from pulse waves, all unit pulse waves which are generated immediately after the end-tidal phase in spontaneous respiration in the subject, the presently disclosed subject matter is not limited to this. Alternatively, a unit pulse wave may be eliminated from pulse waves based on the variation rate of the amplitude of the unit pulse wave.

In the alternative, the eliminator 72 measures the amplitudes of unit pulse waves which are generated after the end-tidal phase of the spontaneous respiration in the subject. The measurement of the amplitudes is performed by measuring the amplitudes of unit pulse waves included in a predetermined time period (for example, one cycle of respiration immediately after the end-tidal phase). The eliminator 72 compares the measured amplitudes of unit pulse waves with the average amplitude of unit pulse waves included in a predetermined time period (for example, one minute), and eliminates a unit pulse wave having a variation rate which is equal to or larger than a predetermined value (for example, 50%) with respect to the average amplitude. The predetermined time period when the average amplitude of unit pulse waves is calculated may be, for example, a time period of one cycle of respiration in which spontaneous respiration, a change of the body posture, a body motion, arrhythmia, and the like do not occur in the subject.

According to the configuration, it is possible to eliminate unit pulse waves which are generated in the predetermined time period after the end-tidal phase of spontaneous respiration, and which have a large variation rate. Therefore, the respiratory variation can be obtained further accurately.

Although, in the embodiment, for example, the PPV is used as the value indicating the respiratory variation as illustrated in Fig. 2, another value such as the SPV may be used.

The pulse wave analyzer of the presently disclosed subject matter includes: a pulse wave acquirer which is configured to acquire a pulse wave of a subject; a respiration information acquirer which is configured to acquire respiration information of the subject; and an analyzing section which includes: a detector which, based on the respiration information, is configured to detect that spontaneous respiration is generated, and to detect an end-tidal phase of the spontaneous respiration; and an eliminator which is configured to eliminate a unit pulse wave that, in the pulse wave, is generated immediately after the end-tidal phase of the spontaneous respiration, from the pulse wave, wherein the analyzing section is configured to calculate a respiratory variation from the pulse wave from which the unit pulse wave has been eliminated.

There is medical knowledge that, in a unit pulse wave which is generated immediately after the end-tidal phase of spontaneous respiration, the waveform is disturbed by abnormal variation of the intrathoracic pressure. According to the above-described configuration, when the respiratory variation is to be calculated, a unit pulse wave which is generated immediately after the end-tidal phase of spontaneous respiration is eliminated. When the respiration of the subject is assisted, for example, by using an artificial respirator, therefore, the respiratory variation which is an index for knowing the circulatory dynamics in the subject can be accurately obtained.

According to the pulse wave analyzer of the presently disclosed subject matter, the accuracy of the calculation of the respiratory variation which is an index for knowing the circulatory dynamics in the subject can be improved.

**Claims**

1.    A pulse wave analyzer (1) comprising:

a pulse wave acquirer (2) which is configured to acquire a pulse wave of a subject;
a respiration information acquirer (3) which is configured to acquire respiration information of the subject; and
an analyzing section (4),
**characterized in that** said analyzing section includes :

a detector (71) which, based on the respiration information, is configured to detect that spontaneous respiration is generated, and to detect an end-tidal phase of the spontaneous respiration; and
an eliminator (72) which is configured to eliminate a unit pulse wave corresponding to one heart beat that, in the pulse wave, is generated immediately after the end-tidal phase of the spontaneous respiration, from the pulse wave, wherein

the analyzing section (4) is configured to calculate a respiratory variation from the pulse wave from which the unit pulse wave has been eliminated.

**Patentansprüche**

1.    Pulswellenanalysator (1), umfassend:

einen Pulswellenerfasser (2), der dazu ausgestaltet ist, eine Pulswelle eines Subjekts zu erfassen;
einen Atmungsinformationserfasser (3), der dazu ausgestaltet ist, Atmungsinformationen des Subjekts zu erfassen; und
einen Analyseabschnitt (4),
**dadurch gekennzeichnet, dass** der Analyseabschnitt einschließt:

einen Detektor (71), der dazu ausgestaltet ist, basierend auf den Atmungsinformationen zu detektieren, dass Spontanatmung erzeugt wird, und eine Endatemzugsphase der Spontanatmung zu detektieren; und
einen Eliminator (72), der dazu ausgestaltet ist, eine einem Herzschlag entsprechende Einheitspulswelle, die unmittelbar nach der Endatemzugsphase der Spontanatmung in der Pulswelle erzeugt wird, aus der Pulswelle zu eliminieren, wobei

der Analyseabschnitt (4) dazu ausgestaltet ist, aus der Pulswelle, aus der die Einheitspulswelle eliminiert wurde, eine Atmungsschwankung zu berechnen.

**Revendications**

1.    Analyseur d'onde de pouls (1) comprenant :

un dispositif d'acquisition d'onde de pouls (2) qui est configuré pour acquérir une onde de pouls d'un sujet ;
un dispositif d'acquisition d'informations de respiration (3) qui est configuré pour acquérir des informations de respiration du sujet ; et
une section d'analyse (4) ;
**caractérisé en ce que** ladite section d'analyse comprend :

un détecteur (71) qui, sur la base des informations de respiration, est configuré pour détecter qu'une respiration spontanée est produite, et pour détecter une phase de fin d'expiration de la respiration spontanée ; et

un dispositif d'élimination (72) qui est configuré pour éliminer une onde de pouls unitaire correspondant à un battement cardiaque qui, dans l'onde de pouls, est produite immédiatement après la phase de fin d'expiration de la respiration spontanée, à partir de l'onde de pouls, dans laquelle

la section d'analyse (4) est configurée pour calculer une variation respiratoire à partir de l'onde de pouls à partir de laquelle l'onde de pouls unitaire a été éliminée.

FIG. 1

*FIG. 2*

ONE RESPIRATION PERIOD

AIRWAY
PRESSURE

<INSPIRATION>          <EXPIRATION>

PULSE PRESSURE
VARIATION OF
ARTERIAL
PRESSURE

$PP_{max}$          $PP_{min}$

FIG. 3

*FIG. 4*

```
                    ( START )
                        │
                        ▼
        ┌───────────────────────────────────┐
        │  ACQUIRE ARTERIAL PRESSURE AND    │── S101
        │  RESPIRATION INFORMATION OF SUBJECT│
        └───────────────────────────────────┘
                        │
                        ▼
        ┌───────────────────────────────────┐
        │    DETECT END-TIDAL PHASE OF      │── S102
        │    SPONTANEOUS RESPIRATION        │
        └───────────────────────────────────┘
                        │
                        ▼
        ┌───────────────────────────────────┐
        │ ELIMINATE UNIT PULSE WAVE GENERATED│
        │    IMMEDIATELY AFTER END-TIDAL    │── S103
        │  PHASE OF SPONTANEOUS RESPIRATION │
        └───────────────────────────────────┘
                        │
                        ▼
        ┌───────────────────────────────────┐
        │ CALCULATE VALUE OF RESPIRATORY VARIATION│── S104
        │  BASED ON PULSE WAVE AFTER ELIMINATION │
        └───────────────────────────────────┘
                        │
                        ▼
        ┌───────────────────────────────────┐
        │ DISPLAY VALUE OF RESPIRATORY VARIATION,│── S105
        │    ETC. ON DISPLAYING SECTION     │
        └───────────────────────────────────┘
                        │
                        ▼
        ┌───────────────────────────────────┐
        │     WHEN ANALYSIS RESULT          │── S106
        │   IS ABNORMAL, NOTIFY THIS        │
        └───────────────────────────────────┘
                        │
                        ▼
                    (  END  )
```

FIG. 5

FIG. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011511686 T
- US 2005245830 A1
- EP 2198776 A1
- EP 2263528 A1